# EUROPEAN PATENT APPLICATION

(11) **EP 4 438 599 A1**
(43) Date of publication of application: **02.10.2024**
(21) Application number: 22916639.2
(22) Date of filing: 23.12.2022
(51) Int. Cl.: C07D 307/46, C07D 307/68, C08G 63/672

(54) **FURANDICARBOXYLIC ACID COMPOUND, METHOD FOR PRODUCING FURANDICARBOXYLIC ACID COMPOUND, POLYESTER, AND METHOD FOR PRODUCING POLYESTER**

(30) Priority: 28.12.2021 KR 20210189995; 16.12.2022 KR 20220177249
(71) Applicant: Kolon Industries, Inc., Seoul 07793 (KR)
(72) Inventor: JEONG, Mee Hye, Seoul 07793 (KR); KIM, Jun Yeong, Seoul 07793 (KR); YONG, Da Kyoung, Seoul 07793 (KR); HA, Ji Min, Seoul 07793 (KR); SHIN, Mi, Seoul 07793 (KR); PARK, No Woo, Seoul 07793 (KR); HONG, Choong hee, Seoul 07793 (KR); PARK, Ki Hyun, Seoul 07793 (KR)
(74) Representative: Ter Meer Steinmeister & Partner
(86) International application number: PCT/KR2022/021247
(87) International publication number: WO 2023/128498

(57) **Abstract**

The present invention relates to a method for preparing a furan dicarboxylic acid compound, which includes preparing a hydroxyl furfural compound from a hydroxyl-free furfural compound; and preparing a furan dicarboxylic acid compound by an oxidation reaction of the hydroxyl furfural compound in the presence of a heterogeneous catalyst.

## Description

### [Technical Field]

The present invention relates to a method for preparing a furan dicarboxylic acid compound, a furan dicarboxylic acid compound prepared therefrom, a method for preparing polyester, and polyester prepared therefrom.

### [Background Art]

In order to reduce greenhouse gases, developed countries such as the United States and Europe have been strengthening carbon dioxide emission regulations. Therefore, the demand for biochemical industries, which can lower dependence on existing fossil raw materials but reduce greenhouse gases are increasing. In other words, chemical industries are changing from oil-dependent to bio-dependent.

For example, technologies to replace polyethylene terephthalate (PET), which is mainly used in beverage bottles and food storage containers, with bio-PET are being actively researched.

Dicarboxylic acid aromatic heterocyclic compounds useful as monomers in the production of bio-PET are derivatives usually prepared by oxidizing 5-hydroxymethyl furfural (HMF). Since the 5-hydroxymethyl furfural (HMF) is obtained from sugar, it is a derivative of a raw material widely available in nature.

For reference, in an oxidation reaction for 5-hydroxymethyl furfural, as shown in Reaction Scheme 1, an aromatic heterocyclic compound of monocarboxylic acid or dicarboxylic acid including 2,5-furan dicarboxylic acid (furan dicarboxylic acid, FDCA) and 2-carboxy-5-formyl furan (FFCA) can be obtained as the main product.

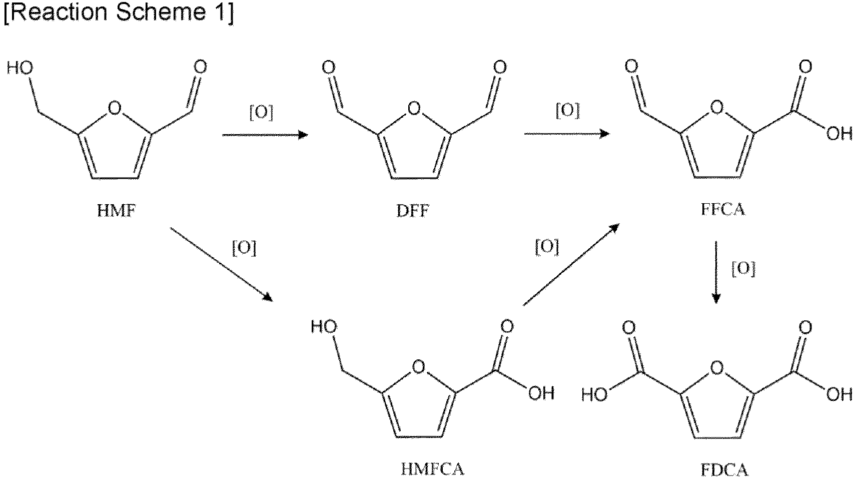

Processes for oxidation of 5-hydroxymethyl furfural by which aromatic heterocyclic compounds of the monocarboxylic acids or dicarboxylic acids can be obtained as main products are known in the literature. For example, U.S. Patent No. 4,977,283 (Hoechst) describes a process for the oxidation of hydroxymethyl furfural carried out by a metal catalyst belonging to the platinum group in an aqueous environment.

Additionally, U.S. Patent publication No. 2008/0103318 (Battelle) describes a method for oxidation of 5-hydroxymethyl furfural promoted by platinum supported on a carrier.

However, the above methods of oxidation of 5-hydroxymethyl furfural have high manufacturing costs and are performed as a batch reaction. This batch reaction requires time that is substantially unrelated to the reaction, such as inputting raw materials, heating to the reaction temperature, and discharging reaction products, and has the problem of lowering the productivity of the reactor.

In addition, in order to repeatedly use the catalyst, a separation process of the catalyst from products is required, ans also, there is a problem of premature decrease in catalyst activity due to changes in temperature, etc.

On the other hand, 2,5-furan dicarboxylic acid prepared by oxidation of 5-hydroxymethyl furfural, which is a compound with low thermal stability, may cause thermal discoloration such as yellowing, browning, and the like through an esterification process with a diol component to prepare polyester, wherein 2-carboxyl-5-formyl furan generated during the oxidation of 5-hydroxymethyl furfural acts as a main factor of discoloring the polyester.

### [Prior Art]

### [Patent Document]

U.S. Patent No. 4,977,283 (December 11, 1990)
U.S. Patent Publication No. 2008-0103318 (May 1, 2008)

### [Disclosure]

### [Technical Problem]

In an embodiment, the purpose is to provide a method for preparing a furan dicarboxylic acid compound which capable of preparing polyester with a higher molecular weight, while preparing a furan dicarboxylic acid compound using a heterogeneous catalyst, discoloration of polyester can be prevented by removing impurities that cause discoloration of polyester.

Another embodiment is to provide a furan dicarboxylic acid compound prepared by the method for preparing a furan dicarboxylic acid compound.

Another embodiment is to provide a method for preparing polyester using a furan dicarboxylic acid compound.

Another embodiment is to provide polyester prepared by the method for preparing polyester.

### [Technical Solution]

According to an embodiment, a method for preparing a furan dicarboxylic acid compound includes preparing a hydroxyl furfural compound from a hydroxyl-free furfural compound, and preparing a furan dicarboxylic acid compound by an oxidation reaction of the hydroxyl furfural compound in the presence of a heterogeneous catalyst.

The hydroxyl-free furfural compound may include chloromethyl furfural (CMF), 2,5-diformylfuran (DFF), 5-acetoxymethylfurfural (AMF), or a mixture thereof.

The hydroxyl furfural compound may include 5-hydroxymethyl furfural (HMF), 5-hydroxymethyl furfural ester, 5-hydroxymethyl furfural ether, or a mixture thereof.

The preparing of a hydroxyl furfural compound may include performing a conversion reaction of a solution including the hydroxyl-free furfural compound and a solvent at 20 °C to 90 °C for 1 minute to 60 minutes while stirring at 100 rpm to 500 rpm.

After the conversion reaction, using an extraction solvent including ethyl acetate (EA), methyl isobutyl ketone, diethyl ether, chloroform, water, or a mixture thereof, a hydroxyl furfural compound can be extracted.

The extraction solvent can be removed by volatilization while reducing a pressure to 10 Torr to 40 Torr at 5 °C to 30 °C.

The method for preparing the furan dicarboxylic acid compound may further include adding a solvent to the prepared hydroxyl furfural compound and then filtering to remove humin.

The oxidation reaction can be accomplished by contacting a solution including the hydroxyl furfural compound and a solvent with an oxidizing agent in the presence of a solid base and a heterogeneous catalyst.

The heterogeneous catalyst may include an active metal including Fe, Ni, Ru, Rh, Pd, Os, Ir, Au, Pt, an alloy thereof, or a mixture thereof, and a carrier supporting the active metal.

The heterogeneous catalyst may be included in an amount of 600 mol or less based on the active metal in the catalyst with respect to 1 mol of the hydroxyl furfural compound.

The solid base may include MgO, CaO, CaCO₃, hydrotalcite, or a mixture thereof.

The solid base may be included in an amount of 10 to 40 parts by weight based on 100 parts by weight of the hydroxyl furfural compound.

The oxidizing agent may include oxygen, air, or a mixture thereof.

The solvent may include water, acetonitrile, acetone, dimethylformamide, dimethyl sulfoxide, ethanol, methanol, t-butyl hypochlorite, or a mixture thereof.

The solvent may be included in an amount of 1000 to 9000 parts by weight based on 100 parts by weight of the hydroxyl furfural compound.

The oxidation reaction may be performed at a pressure of 6 bar to 50 bar and a temperature of 80 °C to 200 °C for 1 hour to 24 hours.

A product of the oxidation reaction may include 2,5-furanedicarboxylic acid (FDCA) and 5-formylfurancarboxylic acid (FFCA).

The method for preparing the furan dicarboxylic acid compound may further include a purification step of removing 5-formylfurancarboxylic acid by contacting the product of the oxidation reaction with hydrogen in the presence of a heterogeneous reduction catalyst and a solvent.

The purification step may be performed at 5 bar to 15 bar and 100 °C to 200 °C for 1 hour to 10 hours.

The heterogeneous reduction catalyst may include an active metal including Cu, Ni, Co, Pd, Pt, Ru, Ag, Au, Rh, Os, Ir, an alloy thereof, or a mixture thereof, and a carrier supporting the active metal.

The method for preparing the furan dicarboxylic acid compound may further include a metal purification step of removing metal components from the product of the oxidation reaction.

The metal component may include Fe, Cr, Ni, Cu, Zn, Mo, Ti, Cd, Co, Mn, Na, Mg, K, Ca, Al, As, Sb, Pb, Sn, B, Si, or a mixture thereof.

The metal purification step can be accomplished using an ion filter.

According to another embodiment, a furan dicarboxylic acid compound is provided, which is prepared according to a method for producing the furan dicarboxylic acid compound and includes less than 200 ppm of metal components based on a total weight.

The furan dicarboxylic acid compound may include 3 wt% or less of humin based on a total weight.

The furan dicarboxylic acid compound may include 5-formylfurancarboxylic acid (FFCA), hydroxymethylfurancarboxylic acid (HMFCA), tetrahydrofuran-2,5-dicarboxylic acid (THFDCA), or a mixture thereof in an amount of 10 wt% or less based on a total weight.

According to another embodiment, a method for preparing polyester includes preparing polyester by subjecting a furan dicarboxylic acid compound and a diol compound to an esterification reaction and then performing a polymerization reaction.

According to another embodiment, provided is a polyester that includes a repeating unit represented by Chemical Formula 7, includes less than 200 ppm of metal components based on a total weight, and has a b* value of 14 or less according to the CIE1976 L*a*b* colorimeter.

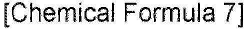

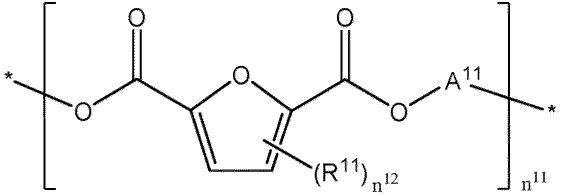

In Chemical Formula 7, A¹¹ is a linear or branched divalent hydrocarbon group having 1 to 15 carbon atoms, R¹¹ is a halogen group, a hydroxy group, an alkoxy group, or an alkyl group, n¹¹ is the number of repetitions of the repeating unit, and n¹² is an integer of 0 to 2.

The metal may include Fe, Cr, Ni, Cu, Zn, Mo, Ti, Cd, Co, Mn, Na, Mg, K, Ca, Al, As, Sb, Pb, Sn, B, Si, or a mixture thereof.

The polyester may include less than 3 wt% of humin based on a total weight.

The polyester may include 5-formylfurancarboxylic acid (FFCA), hydroxymethylfurancarboxylic acid (HMFCA), tetrahydrofuran-2,5-dicarboxylic acid (THFDCA), or a mixture thereof in an amount of less than 10 wt% based on a total weight.

The polyester may have an L* value of 93 or more, an a* value of -0.5 to 0, and a b* value of 14 or less according to the CIE1976 L*a*b* colorimeter.

### [Advantageous Effects]

In the method for preparing a furan dicarboxylic acid compound according to an embodiment, while preparing a furan dicarboxylic acid compound using a heterogeneous catalyst, discoloration of polyester may be prevented by removing impurities that cause discoloration of polyester and a polyester with a higher molecular weight can be prepared.

The polyester according to another embodiment has improved color (yellowness), and as the molecular weight increases, the molecular weight dispersion range narrows, resulting in excellent strength and uniform molecular weight.

### [Description of the Drawings]

FIG. 1 is a graph showing the results of analysis of 2,5-furan dicarboxylic acid prepared in Example 1 using high-performance liquid chromatography (HPLC).
FIG. 2 is a graph showing the results of analysis of 2,5-furan dicarboxylic acid prepared in Comparative Example 1 using high-performance liquid chromatography (HPLC).
FIG. 3 is a graph showing the results of analysis of 2,5-furan dicarboxylic acid prepared in Comparative Example 2 using high-performance liquid chromatography (HPLC).
FIG. 4 is a graph showing the results of analysis of 2,5-furan dicarboxylic acid prepared in Comparative Example 3 using high-performance liquid chromatography (HPLC).
FIG. 5 is a graph showing the results of analysis of 2,5-furan dicarboxylic acid prepared in Comparative Example 4 using high-performance liquid chromatography (HPLC).

### [Mode for Invention]

The advantages and features of the present disclosure and the methods for accomplishing the same will be apparent from the embodiments described hereinafter. However, the embodiments should not be construed as being limited to the embodiments set forth herein. Unless otherwise defined, all terms (including technical and scientific terms) used herein have the same meaning as commonly understood by one of ordinary skill in the art. The terms defined in a generally-used dictionary may not be interpreted ideally or exaggeratedly unless clearly defined. In addition, unless explicitly described to the contrary, the word "comprise," and variations such as "comprises" or "comprising," will be understood to imply the inclusion of stated elements but not the exclusion of any other elements. Further, the singular includes the plural unless mentioned otherwise.

A method for preparing a furan dicarboxylic acid compound according to according to an embodiment includes a conversion reaction step of preparing a hydroxyl furfural compound from a hydroxyl-free furfural compound, and an oxidation reaction step of oxidizing the hydroxyl furfural compound in the presence of a heterogeneous catalyst to prepare a furan dicarboxylic acid compound.

The hydroxyl-free furfural compound may be an aromatic heterocyclic compound represented raw material by Chemical Formula 1.

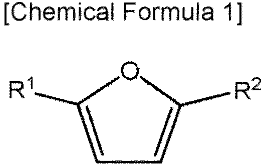

The aromatic heterocyclic compound raw material represented by Chemical Formula 1 is a compound converted from wood and is the first compound used in the production of furan dicarboxylic acid compounds. For example, the aromatic heterocycle may include furan, thiophene, pyrrole, or a combination thereof.

In Chemical Formula 1, one of R¹ and R² is an aldehyde group, and the other is a hydrogen group, a hydroxy group, an aldehyde group, acetoxy group, a halogen, a substituted or unsubstituted C₁-C₂₀ alkyl group, or a substituted or unsubstituted C₁-C₂₀ alkoxy group. That is, in Chemical Formula 1, when R¹ is an aldehyde, R² may be a hydrogen group, a hydroxy group, an aldehyde group, acetoxy group, a halogen, a substituted or unsubstituted C₁-C₂₀ alkyl group, or a substituted or unsubstituted C₁-C₂₀ alkoxy group. Additionally, as an example, the C₁-C₂₀ alkyl group or C₁-C₂₀ alkoxy group may be unsubstituted or substituted with one or more halogen atoms.

For example, the hydroxyl-free furfural compound may include a compound represented by Chemical Formula 2 to Chemical Formula 4, or a combination thereof. That is, the hydroxyl-free furfural compound may include chloromethyl furfural (CMF), 2,5-diformylfuran (DFF), and 5-acetomethylfurfural (AMF), or a mixture thereof.

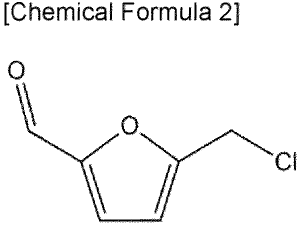

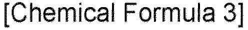

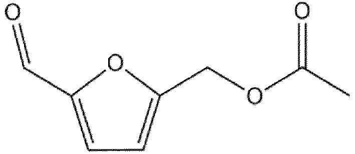

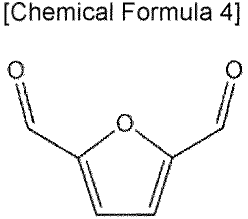

The hydroxyl furfural compound may include 5-hydroxymethyl furfural (HMF), 5-hydroxymethyl furfural ester, 5-hydroxymethyl furfural ether, or a mixture thereof, and 5-hydroxymethyl furfural may be for example represented by Chemical Formula 5.

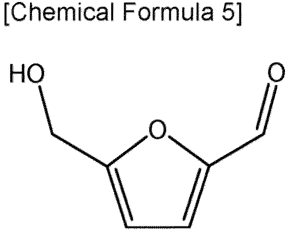

In the conversion reaction step, the hydroxyl furfural compound is prepared from the hydroxyl-free furfural compound.

As an example, the conversion reaction can be accomplished by mixing with a solvent and then stirring.

The solvent may include water, acetonitrile, acetone, dimethylformamide, dimethyl sulfoxide, ethanol, methanol, t-butyl hypochlorite, or a mixture thereof, and for example, when water is used, the hydroxyl-free furfural compound can be converted to 5-hydroxymethylfurfural by donating OH-. In addition, when the hydroxyl-free furfural compound is chloromethylfurfural, it does not dissolve well in water due to hydrophobicity, so that it can be made to dissolve well in water by mixing some polar organic solvents.

Stirring of the conversion reaction may be between 100 rpm and 500 rpm, for example between 150 rpm and 350 rpm. If the stirring in the conversion reaction is less than 100 rpm, the hydroxyl-free furfural compound may not be sufficiently stirred in the solvent, which may lead to difficulties in conversion to the hydroxyl furfural compound. If the speed exceeds 500 rpm, huminization may proceed due to an excessive temperature rise at the contact surface between the stirrer and the reactant due to frictional heat with the solvent of the stirrer, which may cause a decrease in yield.

The conversion reaction may be performed at 20 °C to 90 °C, for example 40 °C to 90 °C. If the temperature of the conversion reaction is less than 20 °C, the reaction may not proceed, and if it exceeds 90 °C, self-polymerization occurs due to high temperature after conversion to a hydroxyl furfural compound, resulting in the production of dimers, trimers, and humins, which may decrease the yield.

The conversion reaction may be performed from 1 minute to 60 minutes, for example from 2 minutes to 30 minutes. If the conversion reaction time is less than 1 minute, the reaction may not start, and if it exceeds 60 minutes, dimer, trimer, and humin may be generated and the yield may decrease.

For example, when the hydroxyl-free furfural compound is chloromethylfurfural, in the conversion reaction, chlorine becomes a chloride ion and meets the hydrogen ion (H⁺) of water to form HCl, and hydroxyl ion (OH-) of water is replaced at the Cl⁻ site to produce 5-hydroxymethylfurfural.

Optionally, after the conversion reaction, a step of extracting the prepared hydroxyl furfural compound may be further included.

Extraction of the prepared hydroxyl furfural compound may be performed using an extraction solvent including ethyl acetate (EA), methyl isobutyl ketone, diethyl ether, chloroform, water, or a mixture thereof.

In addition, optionally, after extracting the hydroxyl furfural compound, removing a solvent used for extraction may be further included.

As an example, the extraction step may further include extracting the hydroxyl furfural compound and then removing the extraction solvent by volatilizing it while reducing the pressure to 10 Torr to 40 Torr at 5 °C to 30 °C.

When removing the extraction solvent, if the temperature is less than 5 °C, the extraction solvent may not volatilize and some may remain in the hydroxyl furfural compound. If it exceeds 30 °C, the internal temperature may rise rapidly depending on the internal pressure, and self-polymerization of the hydroxyl furfural compound may proceed, resulting in a decrease in yield.

It is not easy to maintain the pressure below 10 Torr when removing the extraction solvent, and if the pressure exceeds 40 Torr, the extraction solvent may not be completely volatilized and some may remain in the hydroxyl furfural compound.

Optionally, a step of removing humin from the prepared hydroxyl furfural compound may be further included.

5-Hydroxymethylfurfural is unstable at acidic pH and high temperatures, so that 5-hydroxymethyl furfural polymerizes with each other or with reaction starting materials or reaction by-products, making it soluble or insoluble depending on the chain length and humin may be produced, which causes discoloration of the reaction solution from brown to black. Herein, the reaction starting material may be chloromethyl furfural (CMF), 2,5-diformylfuran (DFF), 5-acetoxymethylfurfural (AMF), or a mixture thereof, and the reaction by-product may be 5-formylfurancarboxylic acid (FFCA), hydroxymethylfurancarboxylic acid (HMFCA), tetrahydrofuran-2,5-dicarboxylic acid (THFDCA), or a mixture thereof.

For example, the step of removing humin can be accomplished by adding a solvent to the hydroxyl furfural compound and then filtering it.

The solvent may include water, acetonitrile, acetone, dimethylformamide, dimethyl sulfoxide, ethanol, methanol, t-butyl hypochlorite, or a mixture thereof.

Filtration may be performed, for example, at room temperature and pressure.

In the oxidation reaction step, the hydroxyl furfural compound is oxidized in the presence of a heterogeneous catalyst to produce a furan dicarboxylic acid compound.

As an example, the oxidation reaction may be performed by contacting a solution including the hydroxyl furfural compound and a solvent with an oxidizing agent in the presence of a solid base and a heterogeneous catalyst.

The heterogeneous catalyst may include an active metal and a carrier supporting the active metal.

The active metal has an activity that contributes to improving product yield when producing a furan dicarboxylic acid compound from a hydroxyl furfural compound.

The active metal may include Fe, Ni, Ru, Rh, Pd, Os, Ir, Au, Pt, or a combination thereof, which may have various oxidation numbers, for example Ru, Pd, Pt, or Au.

The heterogeneous catalyst may be included in an amount of 0.1 wt% to 10 wt%, for example 0.1 wt% to 5 wt%, based on a total weight of the heterogeneous catalyst. If the content of the active metal is less than 0.1 wt%, the oxidation reaction may not proceed, and if it exceeds 10 wt%, the content exceeds that required for the reaction in a continuous process reaction, so that there may be economic loss due to the unnecessary content, and the active metal may not be uniformly deposited on the carrier and the active metal may be clumped together.

The carrier may include a metal oxide, a carbonaceous material, a metal silicate, a metal carbide, or a mixture thereof. The metal oxide may include for example silica, zirconia, alumina, or a mixture thereof. The carbonaceous material may be, for example, carbon black.

For example, the carrier may be a basic metal oxide carrier. The basic metal oxide carrier includes a basic metal oxide that can form a complex by coordinating with the hydroxyl furfural compound.

As an example, a furan dicarboxylic acid compound may be prepared by oxidizing a hydroxyl furfural compound in a polar solvent in the presence of a heterogeneous catalyst. The produced furan dicarboxylic acid compound is a white powder and must exist in a liquid state during the reaction process to not precipitate in the reactor. At this time, the basic metal oxide serves to maintain the product in a liquid phase by coordinating with the furan dicarboxylic acid compound until the reaction is completed.

The basic metal oxides that can form a complex by coordinating with the furan dicarboxylic acid compound may include BaO, Nd₂O₃, Cs₂O, CsX (X is OH, Cl, Br, or I), basic zeolite, a basic composite metal oxide, metal-organic frameworks (MOFs), hydrotalcite, hydroxyapatite, or a combination thereof.

As an example, the basic zeolite is may be a zeolite in which the hydrogen of a high silica beta or mordenite acidic zeolite having a silica/alumina (SiO₂/Al₂O₃) molar ratio of 20 to 1000 is substituted with ions including Na, K, Rb, Cs, Mg, Ca, Sr, Ba, or a combination thereof.

For example, the basic composite metal oxide may include Mg₂Al₂O₄, ZnAl₂O₄, BaTiO₃, ZnTiO₃, MgO-SiO₂, CaO-SiO₂, SrO-SiO₂, BaO-SiO₂, MgO-Al₂O₃, CaO-Al₂O₃, SrO-Al₂O₃, BaO-Al₂O₃, MgO-TiO₂, CaO-TiO₂, SrO-TiO₂, BaO-TiO₂, MgO-ZnO, CaO-ZnO, SrO-ZnO, BaO-ZnO, or a combination thereof.

As an example, the metal-organic framework may include Zr-based MOFs, Mg-based MOFs, Ca-based MOFs, Sr-based MOFs, Ba-based MOFs, or a combination thereof.

As an example, hydrotalcite may include [Mg₄Al₂(OH)₁₂(CO₃) · 4H₂O], [Mg₆Al₂(OH)₁₆(CO₃) · 4H₂O], [Mg₄Zn₂Al₂(OH)₁₆(CO₃) · 4H₂O], [Ca₄Al₂(OH)₁₂(NO₃)₂ · xH₂O], [Ca₆Al₂(OH)₁₆(NO₃)₂ · xH₂O], [Ca₄Zn₂Al₂(OH)₁₆(CO₃) ·4H₂O], or a combination thereof.

An amount of the heterogeneous catalyst used may be, for example, 600 mol or less, or 50 to 600 mol, based on the active metal in the catalyst, based on 1 mol of the hydroxyl furfural compound.

The solid base may include MgO, CaO, CaCO₃, hydrotalcite, or a mixture thereof. The solid base acts as a chelate and dissolves the furan dicarboxylic acid compound in water in salt form. Accordingly, the pH of the oxidation reaction product does not change.

The solid base may be included in an amount of 10 to 40 parts by weight, for example, 15 to 35 parts by weight, based on 100 parts by weight of the hydroxyl furfural compound. If the content of the solid base is less than 10 parts by weight, the furan dicarboxylic acid compound may precipitate as the oxidation reaction progresses, and if it exceeds 40 parts by weight, an excessive amount of HCl aqueous solution is used in the crystallization step of the furan dicarboxylic acid compound, which may lead to environmental pollution and excessive wastewater, thereby increasing the preparation price.

The internal pH during synthesis may be 6 to 8. When proceeding within this pH range, furan dicarboxylic acid compound may be produced but do not precipitate. Precipitation of the furan dicarboxylic acid compound is not a major problem in a batch reactor, but if the furan dicarboxylic acid compound precipitate inside a continuous tubular reactor, it may cause clogging of the nozzle connecting the tubular reactor.

The oxidizing agent may include oxygen, air, or a mixture thereof.

The solvent may include water, acetonitrile, acetone, dimethylformamide, dimethyl sulfoxide, ethanol, methanol, t-butyl hypochlorite, or a mixture thereof, and for example, if water is used, oxygen can be dissolved better.

The solvent may be included in an amount of 1000 to 9000 parts by weight based on 100 parts by weight of the hydroxyl furfural compound. If the solvent content is less than 1000 parts by weight, the hydroxyl furfural compound may not be sufficiently dissolved and the oxidation reaction may not be initiated or the injected oxidizing agent may not be sufficiently dissolved, and if it exceeds 9000 parts by weight, the concentration of the reactant may decrease due to a decrease in the concentration of the hydroxyl furfural compound, which may result in economic loss in the overall process.

The temperature of the oxidation reaction may be 80 °C to 200 °C, for example 100 °C to 180 °C. If the oxidation reaction temperature is less than 80 °C, the reaction may not start, and if it exceeds 200 °C, self-polymerization of the hydroxyl furfural compound may occur and a side reaction may proceed.

The pressure of the oxidation reaction may be 6 bar to 50 bar, for example 8 bar to 30 bar. If the oxidation reaction pressure is less than 6 bar, the yield may decrease because sufficient oxygen is not supplied to produce the furan dicarboxylic acid compound, and if it exceeds 30 bar, the internal temperature may increase and a side reaction may proceed.

The oxidation reaction may be performed for 1 hour to 24 hours, for example 1 hour to 12 hours. If the oxidation reaction time is less than 1 hour, unreaction may occur, and if it exceeds 24 hours, a side reaction may proceed and humin may be produced.

The reaction product obtained through the oxidation reaction step may be a crude composition including the hydroxyl furfural compound and the furan dicarboxylic acid compound.

As an example, the desired furan dicarboxylic acid compound may be 2,5-furan dicarboxylic acid (FDCA) represented by Chemical Formula 6.

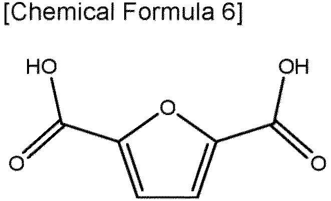

At this time, the reaction product may inevitably include a hydroxyl furfural compound represented by Chemical Formula 1 (for example, a compound represented by Chemical Formula 2 to Chemical Formula 4).

That is, the reaction is an oxidation reaction in the presence of a heterogeneous catalyst using the aforementioned raw materials, and the oxide of the aromatic heterocyclic compound (hereinafter referred to as furan dicarboxylic acid compound or reaction product) obtained from these reactions may include mainly includes 2,5-furandicarboxylic acid (FDCA), but also includes reaction intermediates such as 5-hydroxymethyl-2-furancarboxylic acid (HMFCA) and 5-formylfurancarboxylic acid (FFCA), due to oxidation of the unsaturated group included in the backbone, as shown in Reaction Scheme 1. In addition, tetrahydrofuran-2,5-dicarboxylic acid (THFDCA) generated in the process of purifying 5-hydroxymethyl-2-furancarboxylic acid (HMFCA) and 5-formylfurancarboxylic acid (FFCA) may be included.

Since these by-products generally have a higher melting point than the hydroxyl furfural compound, they are difficult to remove from the reactor in a molten state and require post-processing such as purification or crystallization.

Therefore, optionally, a purification step of removing 5-formylfurancarboxylic acid by contacting the product of the oxidation reaction with hydrogen in the presence of a heterogeneous reduction catalyst and a solvent may be further included. For example, 5-formylfurancarboxylic acid dissolves the reaction product in a solvent, and 5-formylfurancarboxylic acid is removed by breaking the double bond of the furan ring through hydrogenation reaction (hydrogen addition reaction) and dissolving it in water, a solvent, to obtain a furan dicarboxylic acid compound that is insoluble in water.

The heterogeneous reduction catalyst used in the purification step may include an active metal and a carrier supporting the active metal.

The active metal of the heterogeneous reduction catalyst may include Cu, Ni, Co, Pd, Pt, Ru, Ag, Au, Rh, Os, Ir, an alloy thereof, or a mixture thereof.

The carrier may include a metal oxide, a carbonaceous material, a metal silicate, a metal carbide, or a mixture thereof. The metal oxide may include for example silica, zirconia, alumina, or a mixture thereof. The carbonaceous material may be, for example, carbon black.

The amount of the heterogeneous reduction catalyst used may be, for example, 50 to 300 moles based on the active metal in the heterogeneous reduction catalyst, with respect to 1 mole of the product of the oxidation reaction.

The solvent may include water, acetonitrile, acetone, dimethylformamide, dimethyl sulfoxide, ethanol, methanol, t-butyl hypochlorite, or a mixture thereof, and for example, when water is used, the oxidation reaction can be facilitated due to the large amount of oxygen dissolved in the solvent.

The content of the solvent may be 1000 parts by weight to 9000 parts by weight, for example, 2000 parts by weight to 8000 parts by weight, based on 100 parts by weight of the product of the oxidation reaction. If the solvent content is less than 1000 parts by weight, the reaction may not start, and if it exceeds 9000 parts by weight, economic efficiency may be reduced.

The temperature of the purification reaction may be 100 °C to 200 °C, for example 100 °C to 180 °C. If the purification reaction temperature is less than 100 °C, the reaction may not start, and if it exceeds 200 °C, side reactions may proceed.

The pressure of the purification reaction may be 5 bar to 15 bar, for example 6 bar to 13 bar. If the purification reaction pressure is less than 5 bar, double bonds may not be broken due to lack of hydrogen, and if it exceeds 15 bar, side reactions may proceed due to an increase in internal temperature. The purification reaction may be performed for 1 hour to 10 hours, for example 1 hour to 6 hours. If the purification reaction time is less than 1 hour, 5-formylfurancarboxylic acid may not be sufficiently converted to hydroxymethyl furancarboxylic acid, and if it exceeds 10 hours, a side reaction may occur. Optionally, a metal purification step of removing metal components from the product of the oxidation reaction may be further included. As a heterogeneous catalyst is used in the oxidation reaction step, the furan dicarboxylic acid compound may include a metal component, which may cause discoloration of polyester.

The metal component may include Fe, Cr, Ni, Cu, Zn, Mo, Ti, Cd, Co, Mn, Na, Mg, K, Ca, Al, As, Sb, Pb, Sn, B, Si, or a mixture thereof. In particular, the metal component may be a transition metal including Fe, Cr, Ni, Cu, Zn, Mo, Ti, Cd, Co, Mn, or mixtures thereof. The transition metal may affect discoloration during polymer polymerization. Additionally, the transition metal is converted into a metal oxide when heated, and the metal oxide has its own color. For example, when Fe is heated, it easily changes to Fe₂O₃ (Iron(III) oxide), which has a dark brown color. Therefore, discoloration of polyester can be prevented by removing the metal component from the furan dicarboxylic acid compound furan dicarboxylic acid compound.

As an example, the metal purification step may be performed using an ion filter method. The ion filter may have a metal content of less than 200 ppm at room temperature, for example, less than 190 ppm, less than 180 ppm, less than 170 ppm, less than 160 ppm, less than 150 ppm, less than 140 ppm, less than 130 ppm, less than 120 ppm, less than 110 ppm, less than 100 ppm, less than 90 ppm, less than 80 ppm, less than 70 ppm, less than 60 ppm, less than 50 ppm, less than 40 ppm, less than 30 ppm, less than 20 ppm, less than 10 ppm, less than 9 ppm, less than 7 ppm, less than 5 ppm, less than 3 ppm, less than 1 ppm, less than 0.9 ppm, less than 0.8 ppm, less than 0.7 ppm, less than 0.6 ppm, less than 0.5 ppm, less than 0.4 ppm, less than 0.3 ppm, less than 0.2 ppm, less than 0.1 ppm, less than 0.09 ppm, less than 0.08 ppm, or less than or equal to 0.073 ppm. If the content of the metal component is greater than or equal to 200 ppm, discoloration may occur during the polymerization process, which may cause an increase in the b* value of the polyester chip.

Thereafter, the reaction product is titrated with an acid such as HCl, then washed with water (DIW) and subjected to a recrystallization process to obtain a purified furan dicarboxylic acid compound.

A furan dicarboxylic acid compound according to another embodiment is prepared according to the method for preparing a furan dicarboxylic acid compound described above.

As the method for preparing a furan dicarboxylic acid compound further includes a metal purification step, the furan dicarboxylic acid compound may include a metal component, especially a transition metal component, of less than 200 ppm, for example, less than 190 ppm, less than 180 ppm, less than 170 ppm, less than 160 ppm, less than 150 ppm, less than 140 ppm, less than 130 ppm, less than 120 ppm, less than 110 ppm, less than 100 ppm, less than 90 ppm, less than 80 ppm, less than 70 ppm, less than 60 ppm, less than 50 ppm, less than 40 ppm, less than 30 ppm, less than 20 ppm, less than 10 ppm, less than 9 ppm, less than 7 ppm, less than 5 ppm, less than 3 ppm, less than 1 ppm, less than 0.9 ppm, less than 0.8 ppm, less than 0.7 ppm, less than 0.6 ppm, less than 0.5 ppm, less than 0.4 ppm, less than 0.3 ppm, less than 0.2 ppm, less than 0.1 ppm, less than 0.09 ppm, less than 0.08 ppm, or less than 0.073 ppm, or greater than or equal to 0 ppm based on a total weight. If the furan dicarboxylic acid compound includes greater than or equal to 200 ppm of metal components based on a total weight, discoloration may occur during the polymerization process, which may cause an increase in the b* value of the polyester chip.

In addition, as the method for preparing the furan dicarboxylic acid compound further includes the step of removing humin, the furan dicarboxylic acid compound may include humin in an amount of 3 wt% or less, for example, 1 wt% to 3 wt%, based on a total weight. If the furan dicarboxylic acid compound includes greater than 3 wt% of humin based on a total weight, the b* value of the furan dicarboxylic acid compound increases, which acts as a major cause of discoloration during polymerization.

In addition, as the method for preparing furan dicarboxylic acid compound further includes a purification step, the furan dicarboxylic acid compound may include 5-formylfurancarboxylic acid (FFCA), hydroxymethylfurancarboxylic acid (HMFCA), tetrahydrofuran-2,5-dicarboxylic acid (THFDCA), or a mixture thereof in an amount of less than 10 wt%. If the furan dicarboxylic acid compound includes 5-formylfurancarboxylic acid (FFCA), hydroxymethylfurancarboxylic acid (HMFCA), tetrahydrofuran-2,5-dicarboxylic acid (THFDCA), or a mixture thereof in an amount of greater than or equal to 10 wt%, the molecular weight of the polymer may not increase to the target value and chip formation may not occur thereafter.

A method for preparing polyester according to another embodiment includes preparing polyester by subjecting a furan dicarboxylic acid compound and a diol compound to an esterification reaction and then performing a polymerization reaction.

In the esterification reaction step, an oligomer is prepared by reacting an aromatic dicarboxylic acid compound including a furan dicarboxylic acid compound and a diol compound.

The aromatic dicarboxylic acid compound may further include additional aromatic dicarboxylic acid, aliphatic dicarboxylic acid, or a mixture thereof in addition to the furan dicarboxylic acid compound.

The aromatic dicarboxylic acid may be an aromatic dicarboxylic acid having 8 to 20 carbon atoms, for example, an aromatic dicarboxylic acid having 8 to 14 carbon atoms, or a mixture thereof, and may include naphthalene dicarboxylic acids such as isophthalic acid, terephthalic acid, and 2,6-naphthalene dicarboxylic acid, diphenyl dicarboxylic acid, 4,4'-stilbenedicarboxylic acid, 2,5-furan dicarboxylic acid, 2,5-thiophenedicarboxylic acid, etc. The terephthalic acid may include a dicarboxylic acid such as terephthalic acid, alkyl ester thereof (lower alkyl ester with 1 to 4 carbon atoms such as monomethyl, monoethyl, dimethyl, diethyl or dibutyl ester), and/or acid anhydride thereof, and may react with the diol component to form dicarboxylic acid moiety such as terephthaloyl moiety.

The aliphatic dicarboxylic acid may include malonic acid, succinic acid, glutaric acid, 2-methylglutaric acid, 3-methylglutaric acid, adipic acid, pimelic acid, suberic acid, azelaic acid, sebacic acid, undecanedioic acid, and dodecanedioic acid, brassylic acid, tetradecanedioic acid, fumaric acid, 2,2-dimethylglutaric acid, suberic acid, maleic acid, itaconic acid, or maleic acid. In particular, the aliphatic dicarboxylic acid compound may be succinic acid or adipic acid.

The diol compound may be, for example, an aliphatic diol. The aliphatic diol can impart excellent compatibility and elongation to polyesters.

As an example, the aliphatic diol may include linear, branched, or cyclic aliphatic diol components such as ethylene glycol, diethylene glycol, triethylene glycol, propanediol (1,2-propanediol, 1,3-propanediol, etc.), 1,4-butanediol, pentanediol, hexanediol (1,6-hexanediol, etc.), neopentyl glycol (2,2-dimethyl-1,3-propanediol), 1,2-cyclohexanediol, 1,4-cyclohexanediol, 1,2-cyclohexanedimethanol, 1,3-cyclohexanedimethanol, 1,4-cyclohexanedimethanol, and tetramethylcyclobutanediol.

Considering the amount of material lost or unreacted during the esterification reaction, the diol compound may be used in excess of the desired copolymer composition.
For example, in the esterification reaction step, based on 10 parts by mole of the dicarboxylic acid compound, 10 parts by mole to 50 parts by mole, for example 12 parts by mole to 45 parts by mole, 14 parts by mole to 40 parts by mole, 16 parts by mole to 35 parts by mole, or 18 parts by mole to 30 parts by mole of the diol compound may be reacted. If more than 50 parts by mole of the diol compound are used, the esterification reaction rate may be lowered or the physical properties and productivity of the final polyester may be reduced. On the other hand, if less than 10 parts by mole of the diol compound are used, excessive unreacted components of the dicarboxylic acid compound remain, making it impossible to accurately confirm the end point of the esterification reaction. Accordingly, the esterification reaction may proceed for a long time, which may cause thermal discoloration of the final polyester or reduce the physical properties and brightness of the final polyester.

The esterification reaction may be performed under a nitrogen (N₂) atmosphere within a temperature range of 160 °C to 260 °C for 1 hour to 8 hours. The esterification reaction may be appropriately adjusted depending on a mixing ratio of raw materials, specific characteristics of desired polyester, etc. For example, the esterification reaction may be independently performed at greater than or equal to 180 °C, greater than or equal to 170 °C, greater than or equal to 160 °C, or within the range of greater than or equal to 180 °C, less than or equal to 260 °C, less than or equal to 240 °C, or less than or equal to 220 °C. In addition, the esterification reaction may be independently performed for greater than or equal to 1 hour, greater than or equal to 1.5 hours, or greater than or equal to 2 hours and less than or equal to 8 hours, less than or equal to 7 hours, or less than or equal to 6 hours.

If the temperature, pressure, reaction time, etc. of the esterification reaction are respectively below the ranges, properties of the finally prepared polyester may be deteriorated due to a low reaction yield or an insufficient reaction. If the temperature, pressure, reaction time, etc. of the esterification reaction are respectively beyond the ranges, the prepared polyester may be more likely to become yellow, or a depolymerization reaction may proceed, failing in synthesizing polyester in the manufacturing method.

The esterification reaction may be each independently a batch reaction or a continuous reaction.

On the other hand, the esterification reaction may be each independently performed under the presence of an esterification reaction catalyst. In particular, the esterification reaction catalyst may be added to the esterification reaction to improve a reaction rate from the beginning of the reaction and shorten exposure time of polyester to heat.

For example, the esterification reaction catalyst may be a titanium-based catalyst such as titanium oxide, titanium butoxide, or titanium alkoxide.

The esterification reaction catalyst may be used at 10 ppm to 1000 ppm based on central metal atoms of the synthesized polyester. If the content of the esterification reaction catalyst is too small, efficiency of the esterification reaction may be difficult to significantly improve, and an amount of a reactant not participating in the reaction may significantly increase. In addition, if the content of the esterification reaction catalyst is too large, appearance properties of the synthesized polyester may be deteriorated. Furthermore, if the content of the esterification reaction catalyst is too large, the synthesized polyester may become yellow, and appearance properties thereof may be deteriorated.

In the polymerization, the oligomer synthesized in the esterification reaction is polymerized. For example, the polymerization may include pre-polymerization of the oligomer and poly-condensation of the prepolymerized polymer.

In the pre-polymerization, the oligomer may be pre-polymerized to produce a polymer with a higher polymerization degree than the esterification reaction products.

The pre-polymerization, the poly-condensation, or the polymerization including both of them may be performed under the presence of a catalyst.

A polymerization reaction catalyst may be used in an amount of 2 ppm to 100 ppm based on the central metal atoms of the synthesized polyester. If the content of the polymerization reaction catalyst is too small, an amount of a reactant not participating in a reaction may significantly increase, but if the content of the polymerization reaction catalyst is too large, the polymerization reaction catalyst may remain as inert particles, deteriorating properties of the polymer.

On the other hand, a heat stabilizer may be used during the pre-polymerization to minimize thermal discoloration in the pre-polymerization process and the subsequent poly-condensation reaction.

Specifically, the heat stabilizer may include one or more selected from phosphorus-based heat stabilizers including trimethyl phosphonoacetate, triethyl phosphonoacetate, phosphoric acid, phosphorous acid, polyphosphric acid, trimethyl phosphate (TMP), triethyl phosphate, trimethyl phosphine, and triphenyl phosphine.

For example, the heat stabilizer may be added in an amount approximately twice that of the esterification reaction catalyst. If the heat stabilizer is used within the range, thermal discoloration during the pre-polymerization reaction and the poly-condensation reaction may be minimized.

The pre-polymerization may be performed under a temperature control condition (thermal pre-condensation).

Specifically, the pre-polymerization may include increasing a temperature until reaching 220 °C to 260 °C and while maintaining the reached temperature, pre-polymerizing the second esterification reaction products.

In addition, when the temperature is increased, a pressure may be reduced to 0 atm to 0.3 atm, and when the temperature is maintained, the reached pressure also may be maintained.

The pre-polymerization is a step of changing a normal pressure to vacuum in advance before proceeding with a poly-condensation reaction (vacuum) to be described later, which may be a gradual change from normal pressure (1 atm) to vacuum (0 atm). If the pre-polymerization is omitted, in the process of rapidly changing to the vacuum state, the esterification reaction products may be volatilized, causing a bumping phenomenon and reducing a yield of final polyester and also, adversely affecting physical properties thereof.

After the pre-polymerization, the prepolymerized polymer is subjected to a poly-condensation reaction.
The poly-condensation reaction may be performed under a vacuum atmosphere within a temperature range of 220 °C to 300 °C under a pressure of 0.8 torr to 0.4 torr or less for 2 hours to 6 hours.

The poly-condensation reaction may be performed at a higher vacuum level than 0 atm. The vacuum level is measured by using a vacuum gauge, and generally within a range of 0.8 torr to 0.4 torr, the polymerization proceeds.

During the poly-condensation, a poly-condensation reaction catalyst may be used.

The poly-condensation catalyst may be added to the esterification reaction products before initiating the poly-condensation reaction, to a mixture including a diol component and a dicarboxylic acid component before the esterification reaction, or during the esterification reaction.

The polymerization reaction catalyst and the poly-condensation catalyst may each independently be a titanium-based compound, a germanium-based compound, an antimony-based compound, an aluminum-based compound, a tin-based compound, or a mixture thereof.

The titanium-based compound may include tetraethyl titanate, acetyltripropyl titanate, tetrapropyl titanate, tetrabutyl titanate, polybutyl titanate, 2-ethylhexyl titanate, octylene glycol titanate, lactate titanate, triethanolamine titanate, acetylacetonate titanate, ethyl acetoacetic ester titanate, isostearyl titanate, titanium dioxide, a titanium dioxide/silicon dioxide copolymer, a titanium dioxide/zirconium dioxide copolymer, etc.

The germanium-based compound may include germanium dioxide (GeO₂), germanium tetrachloride (GeCl₄), germanium ethyleneglycoxide, germanium acetate, a copolymer thereof, a mixture thereof, etc.

According to another embodiment, a polyester including a repeating unit represented by Chemical Formula 7 is provided.

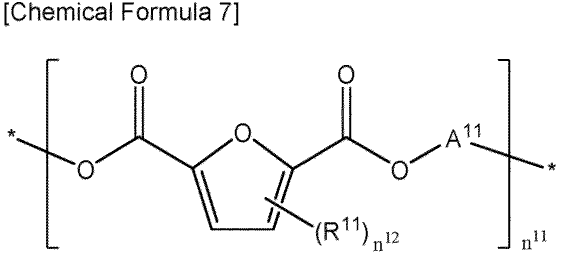

In Chemical Formula 7, A¹¹ are each independently a linear or branched divalent aliphatic hydrocarbon group having 1 to 15 carbon atoms, R¹¹ is a halogen group, a hydroxy group, an alkoxy group, or an alkyl group, n¹¹ is the number of repetitions of each repeating unit, and n¹² is an integer of 0 to 2.

For example, n¹¹ may be appropriately adjusted depending on the molecular weight of the polyester, and for example, n¹¹ may each independently be an integer of 1 to 20.

On the other hand, polyester may include a metal component, especially a transition metal component, of less than 200 ppm, for example, less than 190 ppm, less than 180 ppm, less than 170 ppm, less than 160 ppm, less than 150 ppm, less than 140 ppm, less than 130 ppm, less than 120 ppm, less than 110 ppm, less than 100 ppm, less than 90 ppm, less than 80 ppm, less than 70 ppm, less than 60 ppm, less than 50 ppm, less than 40 ppm, less than 30 ppm, less than 20 ppm, less than 10 ppm, less than 9 ppm, less than 7 ppm, less than 5 ppm, less than 3 ppm, less than 1 ppm, less than 0.9 ppm, less than 0.8 ppm, less than 0.7 ppm, less than 0.6 ppm, less than 0.5 ppm, less than 0.4 ppm, less than 0.3 ppm, less than 0.2 ppm, less than 0.1 ppm, less than 0.09 ppm, less than 0.08 ppm, or less than or equal to 0.073 ppm, or greater than or equal to 0 ppm based on a total weight. If polyester includes greater than or equal to 200 ppm of metal components based on a total weight, discoloration may occur during the polymerization process, which may cause an increase in the b* value of the polyester chip.

In addition, polyester may include humin in an amount of less than or equal to 3 wt%, for example, 1 wt% to 3 wt% based on a total weight. If polyester includes humin in an amount of greater than 3 wt% based on a total weight, a b* value of a polyester chip may be increased.

In addition, polyester may include 5-formylfurancarboxylic acid (FFCA), hydroxymethylfurancarboxylic acid (HMFCA), tetrahydrofuran-2,5-dicarboxylic acid (THFDCA), or a mixture thereof in an amount of less than10 wt% based on a total weight. If polyester includes 5-formylfurancarboxylic acid (FFCA), hydroxymethylfurancarboxylic acid (HMFCA), tetrahydrofuran-2,5-dicarboxylic acid (THFDCA), or a mixture thereof in an amount of greater than or equal to 10 wt% based on a total weight, a molecular weight of a polymer may not rise to a target level, failing in forming a chip.

Polyester may have a number average molecular weight (Mn) of 25,000 g/mol to 65,000 g/mol, for example, 30,000 g/mol to 63,000 g/mol, 35,000 g/mol to 61,000 g/mol, 38,000 g/mol to 60,000 g/mol, or 40,000 g/mol to 58,000 g/mol.

If polyester has a number average molecular weight of less than 25,000 g/mol, it may not only be difficult to process into a film for use as a packaging material, but also a desired modulus may not be achieved. On the contrary, if polyester has a number average molecular weight of greater than 65,000 g/mol, viscosity may be increased, resultantly deteriorating producibility and a yield.

Polyester may have a ratio of weight average molecular weight (Mw)/number average molecular weight (Mn), that is, a molecular weight distribution (MWD) of 1.1 to 3.0, for example, 1.4 to 2.7, 1.6 to 2.5, or 1.79 to 2.4.

If polyester has a molecular weight distribution of less than 1.1, it is difficult to control a process, which leads to a higher defect rate and a higher process cost. On the other hand, if polyester has a molecular weight distribution of greater than 3.0, non-uniform molecular weight distribution may cause defects during manufacturing a product.

Polyester has an L* value of greater than or equal to 93, an a* value of - 0.5 to 0, and a b* value of less than or equal to 14 according to CIE1976 L*a*b* color system, for example, an L* value of greater than or equal to 100, an a* value of -0.5 to 0, and a b* value of less than or equal to 7, less than or equal to 5, less than or equal to 3, less than or equal to 1, or 0 to -1 according to the CIE1976 L*a*b* color system.

For example, colorimeter of polyester is measured as follows. 2 g of a polyester sample is dissolved in 20 ml of hexafluoroisopropanol (HFIP) (0.1 g/ml in HFIP). The sample solution is measured with respect to colorimeter in a Tuartz cell dedicated for solution colorimeter measurement by using CM-3700A made by Konica Minolta.

The higher L*, the closer to white. However, according to use, purpose, etc. of a product to which the polyester is applied, brightness may be controlled within a L* value range of greater than or equal to 93, greater than or equal to 93.5, greater than or equal to 94, or greater than or equal to 94.5 and less than or equal to 100, less than or equal to 99, less than or equal to 98, less than or equal to 97, or less than or equal to 96.

As for the a* value, within the range of less than or equal to 0, the smaller value, the lighter red but the stronger green. However according to use, purpose, etc. of a product to which the polyester is applied, within the a* value range of less than or equal to 0, less than or equal to -0.03, less than or equal to -0.06, or less than or equal to -0.1 and greater than or equal to -0.5, greater than or equal to -0.52, greater than or equal to -0.54, or greater than or equal to -0.56, both red and green levels may be controlled.

As for the b* value, within the range of less than or equal to 14, the smaller value, the lighter yellow but the stronger blue. In particular, according to experimental examples to be described later, all the examples have a b* value of less than or equal to 14, which confirms that thermal discoloration such as yellowing, browning, and the like during the manufacturing process is suppressed. However, according to use, purpose, etc. of a product to which the polyester is applied, within the b* value range of less than or equal to 14, both yellow and blue levels may be controlled.

Polyester may have an intrinsic viscosity of 0.6 dl/g to 2.0 dl/g, for example, 0.7 dl/g to 1.08 dl/g at 25 °C.

In addition, polyester may have an intrinsic viscosity of0.4 dl/g to 1.6 dl/g, for example, 0.6 dl/g to 0.7 dl/g at 35 °C.

For example, polyester may have a glass transition temperature (Tg) of - 40 °C to 50 °C.

### DETAILED DESCRIPTION OF THE EMBODIMENTS

Hereinafter, specific examples of the invention are presented. However, the examples described below are only intended to specifically illustrate or describe the invention, and this should not limit the scope of the invention.

### [Preparation Example 1: Preparation of 2,5-furan dicarboxylic acid (FDCA)] (Example 1)

Chloromethyl furfural (CMF) is mixed with water (H₂O) and then, reacted at about 85 °C for 3 minutes to convert it to 5-hydroxymethyl furfural. The 5-hydroxymethyl furfural (HMF) is extracted with ethyl acetate (EA). The ethyl acetate is volatilized and removed at room temperature under a reduced pressure by using a rotary evaporator, obtaining the 5-hydroxymethyl furfural with a solid content of 100%.

After charging a Ru/C catalyst and a solid base of MgO into a continuous reactor, while supplying a solvent of H₂O and air thereto, the 5-hydroxymethyl furfural is oxidized at 30 bar and 140 °C (WHSV = 0.3 h⁻¹), obtaining 2,5-furan dicarboxylic acid (FDCA) at a yield of about 97%. Herein, a pH change is checked to be maintained at 7. The reason is that the solid base of MgO dissolves the 2,5-furan dicarboxylic acid the form of salts in the water.

After the oxidation is completed, the resultant is titrated with an HCl aqueous solution to crystallize the 2,5-furan dicarboxylic acid and thus obtain the 2,5-furan dicarboxylic acid in the form of powder.

On the other hand, the 2,5-furan dicarboxylic acid is reacted in an autoclave at 150 °C with H₂ at 9 bar for 3 hours to remove 5-formylfurancarboxylic acid and hydroxymethylfurancarboxylic acid, wherein tetrahydrofuran-2,5-dicarboxylic acid generated therefrom is purified, and metal components are removed therefrom by using an ion filter.

### (Comparative Example 1)

2,5-furan dicarboxylic acid is prepared in the same manner as in Example 1 except that the purification process of removing the 5-formylfurancarboxylic acid and the hydroxymethylfurancarboxylic acid and the purification process of removing the metal components are not performed.

### (Comparative Example 2)

2,5-furan dicarboxylic acid is prepared in the same manner as in Example 1 except that the hydroxymethylfurancarboxylic acid alone is removed, but the purification process of removing the metal components is not performed.

### (Comparative Example 3)

2,5-furan dicarboxylic acid is prepared in the same manner as in Example 1 except that the 5-formylfurancarboxylic acid and the hydroxymethylfurancarboxylic acid are removed, but the tetrahydrofuran-2,5-dicarboxylic acid generated during the process of removing the 5-formylfurancarboxylic acid and the hydroxymethylfurancarboxylic acid is not removed, and the purification process of removing the metal components is not performed.

### (Comparative Example 4)

2,5-furan dicarboxylic acid is prepared in the same manner as in Example 1 except that the metal components are not removed.

### [Experimental Example 1: Component analysis of 2,5-furan dicarboxylic acid]

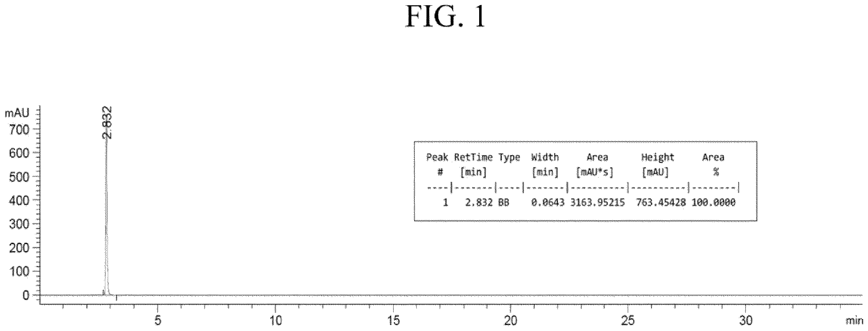

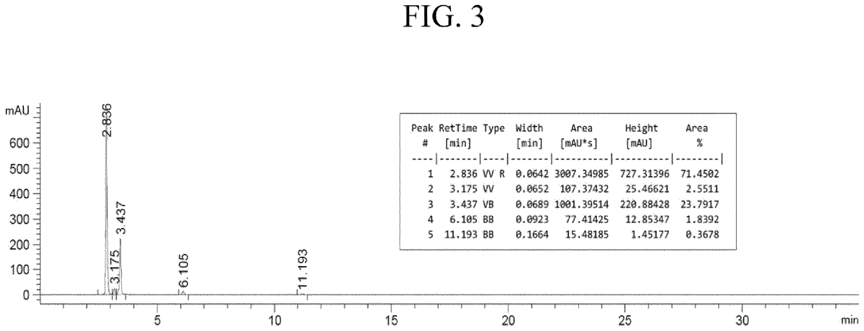

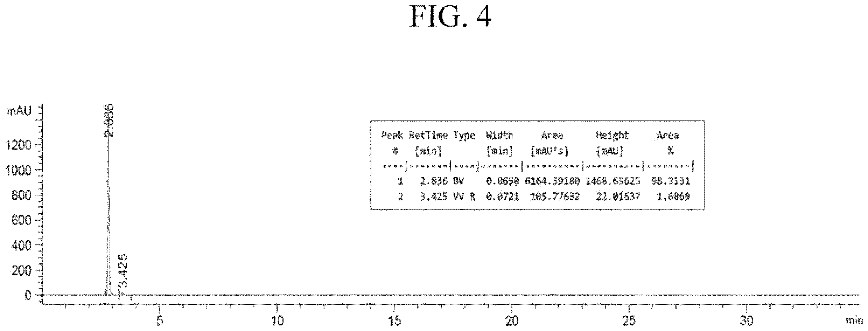

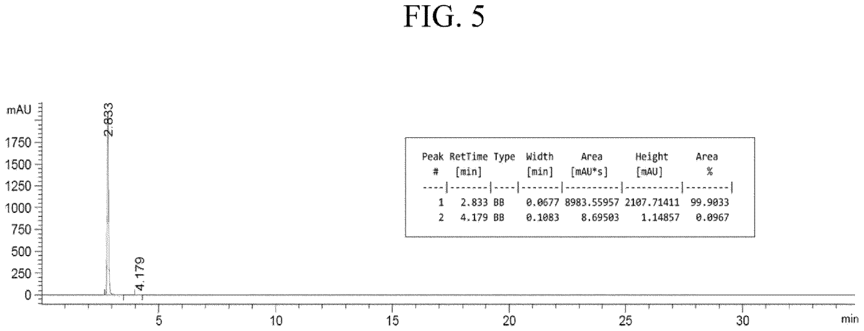

The 2,5-furan dicarboxylic acids according to the examples and the comparative examples are subjected to component analysis by using high performance liquid chromatography (HPLC), and the results are respectively provided in FIGS. 1 to 5.

In addition, the contents of metal components of 2,5-furan dicarboxylic acid prepared in Examples and Comparative Examples are measured by inductively coupled plasma spectroscopy (icp-oes), and the results are summarized in Table 1.

The inductively coupled plasma spectroscopy involves introducing each liquid sample into plasma and then, if light is emitted from a monochromator/multicolor device, a detector is focused to process signals derived therefrom at this time to quantify an elemental composition.

**(Table 1)**

| Metal component (unit: ppm) | λ | Comparative Example 1 | Example 1 | Comparative Example 2 | Comparative Example 4 |
|---|---|---|---|---|---|
| Na | 588.9 | 129.9 | 33.364 | 112.53 | 100.2 |
| Ca | 393.2 | 20 | 20 | 3.2 | 25.132 |
| K | 766.4 | N.D | N.D | 32 | 38.222 |
| Mg | 279.5 | 60.2 | 22.5 | 55.5 | 45.5 |
| Fe | 238.204 | 0.482 | 0.073 | 13.57 | 1.67 |
| Mn | 257.61 | N.D | N.D | 0.174 | N.D |
| Co | 228.616 | N.D | N.D | N.D | N.D |
| Cr | 267.716 | N.D | N.D | N.D | N.D |
| Ni | 221.647 | N.D | N.D | N.D | N.D |
| Cu | 324.754 | N.D | N.D | N.D | N.D |
| Mo | 202.03 | N.D | N.D | N.D | N.D |
| Pb | 246.9 | N.D | N.D | N.D | N.D |
| Ti | 323.452 | N.D | N.D | 10 | N.D |
| Cd | 226.502 | N.D | N.D | N.D | N.D |
| Zn | 213.856 | N.D | N.D | 5 | N.D |
| total | - | 210.582 | 75.937 | 231.974 | 210.724 |

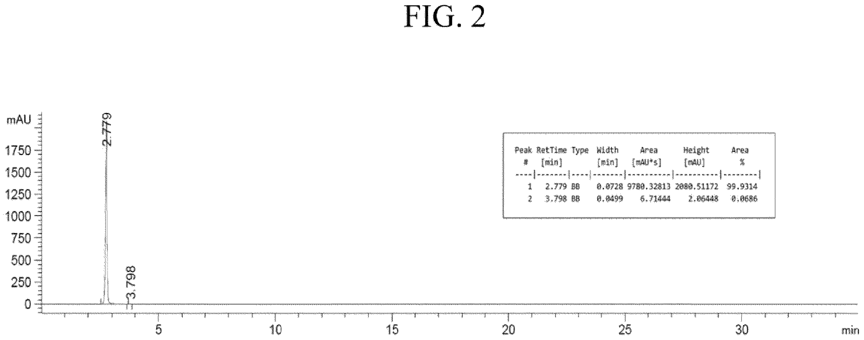

FIG. 1 is the HPLC result after FDCA purification, and FIGS. 2 to 5 are the HPLC results showing the presence of the FFCA peak before FDCA purification. In FIGS. 1 to 5, the highest peak on the left represents FDCA, and a smaller peak to the right represents FFCA. FFCA, if included in an amount of 3% or more, acts end capping during the polyester polymerization to prevent an increase in a molecular and also, works as a major factor in discoloration.

### [Preparation Example 2: Preparation of polyester]

After adding 0.9 mol of 2,5-furan dicarboxylic acid and 1.2 mol of ethylene glycol (EG) to a hopper of a 10 L autoclave reactor, a Ti-based catalyst (material name: titanium isopropoxide) is added at 50 ppm based on Ti-based metal thereto to proceed with an esterification reaction within a temperature range of 210 °C under a nitrogen atmosphere at 2.5 bar for 3 hours.

When the esterification reaction is completed, the internal temperature of the reactor is increased to a temperature range of 240 °C for 1 hour, and a vacuum pump is used to gradually reduce the pressure for 1 hour to an internal pressure of 0.7 Torr or less, and when a load transmitted to a Torque meter of the autoclave reaches 80% Torque, polyester is obtained by draining therefrom.

### [Experimental Example 2: Analysis of polyester]

Each polyester prepared by respectively using the 2,5-furan dicarboxylic acids of Example 1 and Comparative Example 1 is measured through gel permeation chromatography (GPC), and the measurement results are shown in Table 2.

**(Table 2)**

| | Mn | Mw | Mz | PDI |
|---|---|---|---|---|
| Comparative Example 1 | 16160 | 37853 | 72364 | 2.3423 |
| Example 1 | 20678 | 37574 | 54835 | 1.8171 |

Referring to Table 2, the polyester prepared by using the 2,5-furan dicarboxylic acid of Comparative Example 1, in which 5-formylfurancarboxylic acid, etc. are not purified, exhibits PDI (=Mw/Mn) of about 2.34, and the polyester purified by using the 2,5-furan dicarboxylic acid of Example 1, in which 5-formylfurancarboxylic acid, etc. are purified, exhibits reduced PDI (= Mw/Mn) of about 1.81.

In addition, the polyester prepared by using the 2,5-furan dicarboxylic acid of Example 1 exhibits a reduced low molecular peak, Mn shifted toward a high molecular weight, and narrow overall PDI, which confirms that a more monodisperse polymer is obtained.

In addition, the polyesters prepared by respectively using the 2,5-furan dicarboxylic acids Example 1 and Comparative Examples 1 and 4 are measured with respect to a FFCA content, viscosity (IV), and b* value, and the results are shown in Table 3.

**(Table 3)**

| | Comparative Example 1 | Comparative Example 4 | Example 1 |
|---|---|---|---|
| FFCA content (wt%) | 1.68% | 0% | 0% |
| Polyester color | 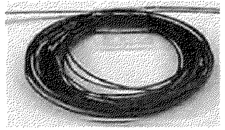 | 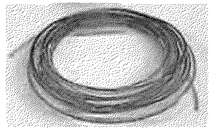 | 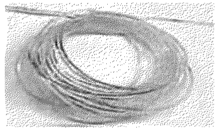 |
| Polymerization time | about 2 hours 30 minutes | about 2 hours 30 minutes | about 2 hours 30 minutes |
| Viscosity (IV) | 0.521 | 0.533 | 0.529 |
| b* | 25 | 17 | 1 |

Referring to Table 3, if a total transition metal content is 200 ppm or less, there is no discoloration of polyester.

Although the exemplified embodiments of the present invention have been described above, but the present invention is not limited thereto but may be implemented with various modifications within the scope of the claims, which also belong to the present invention.

### [Industrial Applicability]

The present disclosure provides a method for preparing polyester with a larger molecular weight by using a heterogeneous catalyst to prepare a furan dicarboxylic acid compound but removing impurities that cause discoloration of polyester therefrom to prevent the discoloration of polyester.

## Claims

1. A method for preparing a furan dicarboxylic acid compound, comprising
preparing a hydroxyl furfural compound from a hydroxyl-free furfural compound, and
preparing a furan dicarboxylic acid compound by an oxidation reaction of the hydroxyl furfural compound in the presence of a heterogeneous catalyst.

2. The method of claim 1, wherein
the hydroxyl-free furfural compound includes chloromethyl furfural (CMF), 2,5-diformylfuran (DFF), 5-acetoxymethylfurfural (AMF), or a mixture thereof, and
the hydroxyl furfural compound includes 5-hydroxymethyl furfural (HMF), 5-hydroxymethyl furfural ester, 5-hydroxymethyl furfural ether, or a mixture thereof.

3. The method of claim 1, wherein
the preparing of the hydroxyl furfural compound includes performing a conversion reaction of a solution including the hydroxyl-free furfural compound and a solvent at 20 °C to 90 °C for 1 minute to 60 minutes while stirring at 100 rpm to 500 rpm.

4. The method of claim 1, wherein
adding a solvent to the prepared hydroxyl furfural compound and then filtering to remove humin is further included.

5. The method of claim 1, wherein
the oxidation reaction is performed by contacting a solution including the hydroxyl furfural compound and a solvent with an oxidizing agent in the presence of a solid base and a heterogeneous catalyst.

6. The method of claim 1, wherein
the heterogeneous catalyst includes an active metal including Fe, Ni, Ru, Rh, Pd, Os, Ir, Au, Pt, an alloy thereof, or a mixture thereof, and a carrier supporting the active metal.

7. The method of claim 5, wherein
the solid base includes MgO, CaO, CaCO₃, hydrotalcite, or a mixture thereof.

8. The method of claim 5, wherein
the solvent includes water, acetonitrile, acetone, dimethyl formamide, dimethyl sulfoxide, ethanol, methanol, t-butyl hypochlorite, or a mixture thereof.

9. The method of claim 1, wherein
the oxidation reaction is performed at a pressure of 6 bar to 50 bar and a temperature of 80 °C to 200 °C for 1 hour to 24 hours.

10. The method of claim 1, wherein
a product of the oxidation reaction includes 2,5-furan dicarboxylic acid (FDCA) and 5-formylfurancarboxylic acid (FFCA), and
a purification step of removing 5-formylfurancarboxylic acid by contacting the product of the oxidation reaction with hydrogen in the presence of a heterogeneous reduction catalyst and a solvent is further included.

11. The method of claim 10, wherein
the purification step is performed at 5 bar to 15 bar and 100 °C to 200 °C for 1 hour to 10 hours.

12. The method of claim 1, wherein
a metal purification step of removing metal components from the product of the oxidation reaction is further included.

13. The method of claim 12, wherein
the metal component includes Fe, Cr, Ni, Cu, Zn, Mo, Ti, Cd, Co, Mn, Na, Mg, K, Ca, Al, As, Sb, Pb, Sn, B, Si, or a mixture thereof.

14. The method of claim 12, wherein
the metal purification step is performed using an ion filter.

15. A furan dicarboxylic acid compound prepared according to method for preparing a furan dicarboxylic acid compound of claim 1,
wherein the furan dicarboxylic acid compound includes less than 200 ppm of metal components based on a total weight.

16. A method for preparing polyester
preparing polyester by subjecting the furan dicarboxylic acid compound of claim 15 and a diol compound to an esterification reaction and then performing a polymerization reaction.

17. A polyester, comprising
a repeating unit represented by Chemical Formula 7,
wherein metal components are included in an amount of less than 200 ppm based on a total weight, and
a b* value according to the CIE1976 L*a*b* colorimeter is 14 or less: 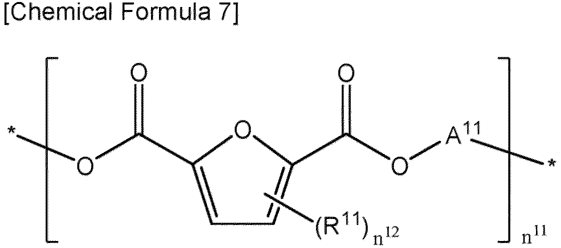
wherein, in Chemical Formula 7,
A¹¹ is a linear or branched divalent hydrocarbon group having 1 to 15 carbon atoms,
R¹¹ is a halogen, a hydroxy group, an alkoxy group, or an alkyl group,
n¹¹ is the number of repetitions of the repeating unit, and
n¹² is an integer of 0 to 2.

18. The polyester of claim 17, wherein
the polyester includes less than 3 wt% of humin based on a total weight.

19. The polyester of claim 17, wherein
the polyester includes 5-formylfurancarboxylic acid (FFCA), hydroxymethylfurancarboxylic acid (HMFCA), tetrahydrofuran-2,5-dicarboxylic acid (THFDCA), or a mixture thereof in an amount of less than 10 wt% based on a total weight.

20. The polyester of claim 17, wherein
the polyester has an L* value of 93 or more, an a* value of -0.5 to 0, and a b* value of 14 or less according to the CIE1976 L*a*b* colorimeter.
